# EUROPEAN PATENT APPLICATION

(11) **EP 4 678 225 A1**
(43) Date of publication of application: **14.01.2026**
(21) Application number: 24196066.5
(22) Date of filing: 23.08.2024
(51) Int. Cl.: A61N 1/40, A61N 2/02, A61B 18/00, A61N 1/06

(54) **NON-INVASIVE HIGH-FREQUENCY TREATMENT SYSTEM**

(30) Priority: 09.07.2024 KR 20240090462
(71) Applicant: Four S Tech Co.,Ltd, Gyeonggi-do 14449 (KR)
(72) Inventor: EOM, Tae Min, 13306 Gyeonggi-do (KR)
(74) Representative: Zacco Sweden AB

(57) **Abstract**

A non-invasive high-frequency treatment system is disclosed. The non-invasive high-frequency treatment system according to one aspect of the present invention includes a conductive material injected into a skin to be treated, and a high-frequency treatment device configured to generate a high-frequency current to transmit an alternative magnetic field to the conductive material, wherein the high-frequency treatment device includes a high-frequency generator configured to output the high-frequency current, a coil part configured to convert the high-frequency current output from the high-frequency generator into the alternative magnetic field, and a controller configured to control the high-frequency generator, and the conductive material generates heat due to the alternative magnetic field generated by the coil part.

## Description

### BACKGROUND

### 1. Field of the Invention

The present invention relates to a non-invasive high-frequency treatment system, and more particularly, to a non-invasive high-frequency treatment system that enables skin treatment (procedure) using high-frequency without inserting a needle into the skin.

### 2. Discussion of Related Art

The human skin is composed of an epidermis layer, a dermis layer, and a subcutaneous fat layer. The epidermis layer prevents the invasion of bacteria and the like and prevents moisture from escaping from the body, and the dermis layer includes fibrous proteins and matrix, such as collagen and elastin and plays a role in supplying nutrients to the epidermis layer. In addition, the subcutaneous fat layer is located below the dermis layer and functions to absorb external pressure or impact and maintain body temperature.

As the skin ages, elasticity gradually decreases and wrinkles tend to form, and the reason for this is that the amounts of collagen, elastin, and matrix in the dermis layer generally decrease, and the amount of moisture decreases.

In order to improve fine lines, pores, aging, scars, and the like on the skin, a high-frequency skin cosmetic method is used, in which an electrode that transmits electrical energy is inserted into the dermis layer and high-frequency energy is applied directly. High-frequency skin cosmetic technology uses a fine needle as an electrode to directly apply electrical energy with a frequency to the human body, and in this case, at the part at which the needle is in contact with the skin, the electrical energy is converted into thermal energy, and the converted thermal energy stimulates the regeneration of collagen and elastin, which are skin growth factors.

However, the conventional high-frequency skin cosmetic method has a problem in that heat is generated in the needle used as an electrode, causing the skin around the needle to heat up, which may lead to side effects such as redness at the treatment site or mild burns on the skin after the procedure.

The related art of the present invention is disclosed in Korean Patent Publication No. 10-1124675 (published on March 19, 2012) entitled "Skin Treatment Device Using High-Frequency."

### SUMMARY OF THE INVENTION

The present invention is directed to providing a non-invasive high-frequency treatment system that enables skin treatment using high-frequency without inserting a needle into the skin.

Objectives to be achieved by the present invention are not limited to the above-described objective(s), and other objective(s), which are not described above, may be clearly understood by those skilled in the art through the following specification.

According to an aspect of the present invention, there is provided a non-invasive high-frequency treatment system includes a conductive material injected into a skin to be treated, and a high-frequency treatment device configured to generate a high-frequency current to transmit an alternative magnetic field to the conductive material, wherein the high-frequency treatment device includes a high-frequency generator configured to output the high-frequency current, a coil part configured to convert the high-frequency current output from the high-frequency generator into the alternative magnetic field, and a controller configured to control the high-frequency generator, and the conductive material generates heat due to the alternative magnetic field generated by the coil part.

In the present invention, the conductive material may include nanoparticles of a material having an electrical conductivity greater than or equal to a predetermined value.

In the present invention, the conductive material may be in the form of at least one of hydrogel and collagen.

In the present invention, the conductive material may convert a current induced by the alternative magnetic field into heat through an eddy current loss and a hysteresis loss.

In the present invention, the heat converted in the conductive material may be transmitted as thermal energy to a portion of a dermis layer in contact with the conductive material, thereby treating the skin.

In the present invention, the coil part may include a substrate, and a coil disposed on the substrate, wherein the substrate may be formed of a non-conductor, and may have one surface serving as a close contact surface in contact with the skin of a user during a procedure and the other surface on which the coil is disposed.

In the present invention, the coil part may be disposed on a front end of a handpiece case.

In the present invention, the controller may change at least one of an intensity, a frequency, and a treatment time of high-frequency according to a condition of the skin to be treated or a treatment site.

In the present invention, the high-frequency treatment device may further include a power supply that supplies power, wherein the power supply may be charged by a wired or wireless charging method.

### BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:
FIG. 1 is a diagram schematically illustrating a configuration of a non-invasive high-frequency treatment system according to one embodiment of the present invention;
FIG. 2 is a diagram schematically illustrating a configuration of a high-frequency treatment device according to one embodiment of the present invention;
FIG. 3 is a perspective view illustrating a handpiece of the high-frequency treatment device according to one embodiment of the present invention;
FIG. 4 is an exemplary view for describing a front end of the high-frequency treatment device according to the present embodiment;
FIG. 5 is an exemplary view for describing a coil part according to one embodiment of the present invention; and
FIG. 6 is an exemplary view for describing a state in which a conductive material according to one embodiment of the present invention outputs thermal energy into a dermis layer.

### DETAILED DESCRIPTION OF EXEMPLARY EMBODIMENTS

Hereinafter, with reference to the accompanying drawings, a non-invasive high-frequency treatment system according to one embodiment of the present invention will be described. In this process, thicknesses of lines and sizes of components shown in the drawings may be exaggerated for clarity and convenience of description.

Further, the terms used herein are merely used for the purpose of describing particular embodiments and are not intended to be limiting of the present invention. As used herein, singular forms are intended to include plural forms as well, unless the context clearly indicates otherwise. In the present application, it will be understood that the terms "comprise," "comprising," "include," and/or "including", when used in this specification, specify the presence of stated features, integers, steps, operations, elements, components and/or groups thereof but do not preclude the presence or addition of one or more other features, integers, steps, operations, elements, components and/or groups thereof. While terms such as "first," "second," and the like may be used to describe various components, such components should not be limited to the above terms. These terms are used only for the purpose of distinguishing one component from another component.

Hereinafter, the embodiments of the present invention will be described in detail with reference to the accompanying drawings. but equal or corresponding components will be referred to as the same reference numerals regardless of drawing signs, and redundant descriptions thereof will be omitted.

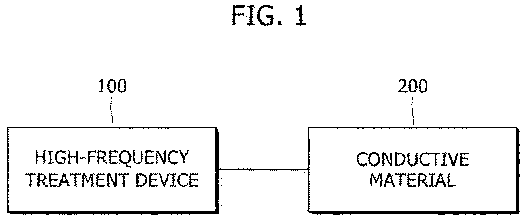

FIG. 1 is a diagram schematically illustrating a configuration of a non-invasive high-frequency treatment system according to one embodiment of the present invention.

Referring to FIG. 1, the non-invasive high-frequency treatment system according to one embodiment of the present invention may include a high-frequency treatment device 100 and a conductive material 200 injected (or inserted) into the skin to be treated.

The conductive material 200 is a material that is injected (or inserted) into the skin to be treated before a treatment (procedure), and may include nanoparticles of a material having an electrical conductivity greater than or equal to a predetermined value.

The conductive material 200 may be in the form of hydrogel, collagen, or the like. The conductive material 200 may be in a highly viscous formulation.

For example, the conductive material 200 may include materials that are harmless to the human body, from among materials, such as silver (105% of the international annealed copper standard (IACS)), copper (100% IACS), gold (70% IACS), and aluminum (61% IACS), which have a high electrical conductivity. In particular, gold nanoparticles have excellent biocompatibility and thus do not cause an immune rejection response, and have high reduction potential and thus are less likely to be present in the body in an ionic form, which makes the gold nanoparticles suitable for use in a patient's body.

The gold nanoparticles processed into nanoparticles may be injected in the form of hydrogel into the skin to be treated (affected area) using a syringe. When the size of the gold nanoparticles is 9 to 10 nm or smaller, the gold nanoparticles can be naturally excreted through the kidneys. For short-term treatments, using a gold nanoparticle hydrogel with a size of 9 to 10 nm or smaller allows the gold nanoparticles to remain in the affected area while the hydrogel state is maintained, enabling multiple procedures (treatments). However, when the size of the gold nanoparticles is 9 to 10 nm or smaller, an energy transfer effect may be weak due to the small size of the gold nanoparticles. In order to maximize the energy transfer effect, the size of the gold nanoparticles should be greater than 10 nm. When the size of the gold nanoparticles is greater than 10 nm, the gold nanoparticles can be permanently used, but are more likely to accumulate in the body rather than being excreted from the body.

The conductive material 200 may generate heat by an alternative magnetic field generated by the high-frequency treatment device 100. That is, since the conductive material 200 includes conductive nanoparticles, a current flows in the conductive material 200 due to the alternative magnetic field generated by the high-frequency treatment device 100, and the conductive material 200 may convert the current into heat through an eddy current loss and a hysteresis loss.

The conductive material 200, which has a high electrical conductivity, generates a swirling current flow, i.e., an eddy current, due to the alternative magnetic field, and the generated eddy current is converted into heat in the conductive material 200, which is referred to as the eddy current loss. The hysteresis loss may refer to an energy loss that occurs when the conductive material 200 is magnetized and demagnetized by the alternative magnetic field.

The conductive material 200 is designed to be injectable (insertable) into the skin at a depth of approximately 0.1 mm to 4 mm, and a current may flow in the conductive material 200 due to an alternative magnetic field generated by a coil part 130 of the high-frequency treatment device 100. In this case, due to the flowing current, an eddy current loss and a hysteresis loss may occur in the conductive material 200. The conductive material 200 can convert a current into heat due to the eddy current loss and the hysteresis loss.

The heat generated in the conductive material 200 may be transmitted as thermal energy to the dermis layer of the skin, with which the conductive material 200 is in contact, allowing the skin to be treated.

The high-frequency treatment device 100 may generate a high-frequency current to transmit an alternative magnetic field to the conductive material 200.

The alternative magnetic field may induce an eddy current in the conductive material 200 including conductors. The eddy current may be converted into heat due to the eddy current loss and the hysteresis loss in the conductive material 200. The heat generated in the conductive material 200 may be transmitted as thermal energy to the dermis layer of the skin in contact with the conductive material 200.

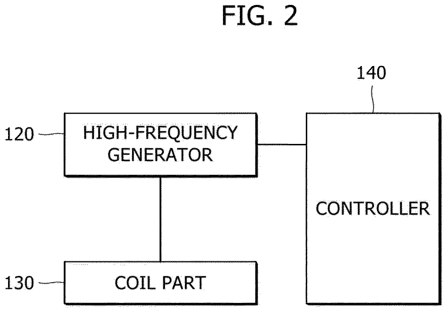

Detailed description of the high-frequency treatment device 100 will be provided with reference to FIG. 2.

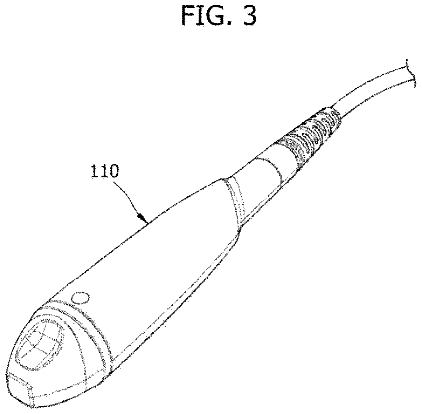

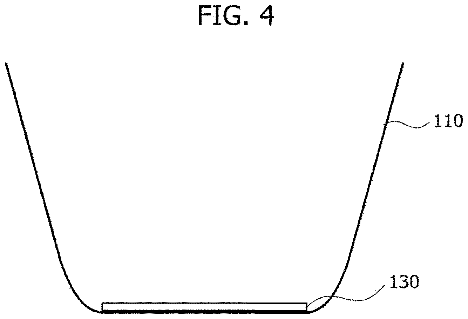

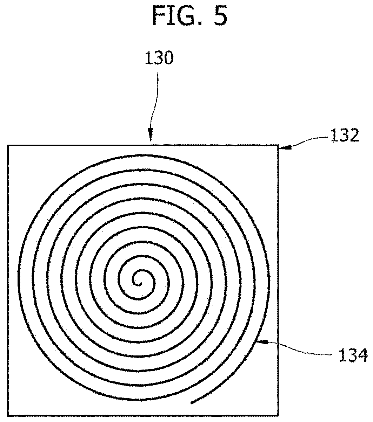

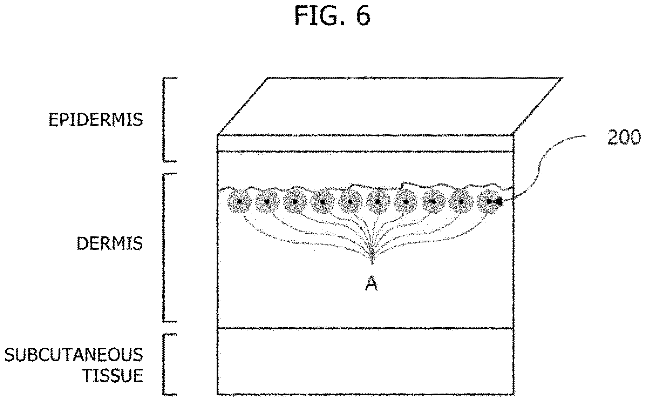

FIG. 2 is a diagram schematically illustrating a configuration of the high-frequency treatment device according to one embodiment of the present invention, FIG. 3 is a perspective view illustrating a handpiece of the high-frequency treatment device according to one embodiment of the present invention, FIG. 4 is an exemplary view for describing a front end of the high-frequency treatment device according to the present embodiment, FIG. 5 is an exemplary view for describing the coil part according to one embodiment of the present invention, and FIG. 6 is an exemplary view for describing a state in which the conductive material according to one embodiment of the present invention outputs thermal energy into the dermis layer.

Referring to FIGS. 2 and 3, the high-frequency treatment device 100 according to one embodiment of the present invention includes a high-frequency generator 120, the coil part 130, and a controller 140. Here, the high-frequency generator 120 and the controller 140 may be provided in a body (not shown) of the high-frequency treatment device 100, and the coil part 130 may be provided on a handpiece case 110 as shown in FIG. 3.

The handpiece case 110 also serves as a handle that a user can grip, and may provide an internal space. The handpiece case 110 has a substantially cylindrical shape, and may have an internal space in which the coil part 130 is installed. Such a handpiece case 110 may be formed of an insulating material such as plastic or the like.

A front end of the handpiece case 110 may be a part of the handpiece case 110 that comes into contact with the skin.

The high-frequency generator 120 may receive power and output a high-frequency current.

The high-frequency generator 120 may include an inverter (not shown) that converts direct current (DC) power into alternating current (AC) power in the range of 100 kHz to 10 MHz to generate induced electromotive force. Specifically, the high-frequency generator 120 may include an input filter circuit (not shown), a primary rectifier circuit (not shown), a power factor correction circuit (not shown), a switching circuit (not shown), an isolation transformer (not shown), a secondary rectifier circuit (not shown), an inverter circuit (not shown), and an output common filter (not shown). The input filter circuit may block noise from the input AC power, the primary rectifier circuit and the power factor correction circuit may improve a power factor while maintaining an input voltage range at 85 V to 264 V, the switching circuit may switch the DC power input from the power factor correction circuit and transmit the DC power as AC to the isolation transformer, the isolation transformer may isolate a primary side from a secondary side while adjusting an output voltage, the secondary rectifier circuit may rectify the AC power input from the isolation transformer into DC, the inverter circuit may adjust a frequency to be transmitted to the coil part 130, and the output common filter may remove common mode noise contained in the output power before transmitting the output power to the coil part 130.

The high-frequency generator 120 may be connected to each of the coil part 130 and positive (+) and negative (-) terminals, and may receive power and transmit the power to the coil part 130. The high-frequency generator 120 may cause the high-frequency current to flow into the coil part 130.

The coil part 130 may convert the high-frequency current output from the high-frequency generator 120 into an alternative magnetic field. That is, the coil part 130 may convert an AC current input from the high-frequency generator 120 into an alternative magnetic field with energy.

For example, the coil part 130 may convert an AC current in the range of 100 kHz to 10 MHz transmitted from the high-frequency generator 120 into an alternative magnetic field through a coil 134.

The coil part 130 may be disposed on the front end of the handpiece case 110 as shown in FIG. 4. That is, the coil part 130 may be disposed on the part in contact with the skin.

The coil part 130 may include the coil 134 and a substrate 132 on which the coil 134 is disposed, as shown in FIG. 5.

The coil 134 may be spirally disposed on the substrate 132.

The coil 134 is located on the substrate 132, and may be located between the substrate 132 and a close contact surface of the handpiece case 110 in contact with the skin. When the coil part 130 is located closest to the skin, the maximum energy can be transmitted.

One surface of the substrate 132 may be the close contact surface that is in contact with the user's skin during a procedure, and the coil 134 may be disposed on the other surface of the substrate 132.

The substrate 132 may be fabricated using a non-metallic material, such as rubber, silicone, plastic, or the like, so as not to affect the high-frequency current flowing through the coil 134.

The coil part 130 is disposed at the front end of the handpiece case 110 and comes into contact with the user's skin.

When high-frequency is generated in a state in which the skin is pressed by the close contact surface in contact with the user's skin, the coil part 130 may convert a high-frequency current output from the high-frequency generator 120 into an alternative magnetic field.

The alternative magnetic field may induce an eddy current in the conductive material 200. Thus, due to the alternative magnetic field generated by the coil part 130, the eddy current flows in the conductive material 200 injected into the skin. The conductive material 200 may convert a current into heat due to an eddy current loss and a hysteresis loss caused by the eddy current.

The conductive material 200 allows a current to flow due to the alternative magnetic field, and may generate heat due to an eddy current loss and a hysteresis loss caused by the current. The heat generated by the conductive material 200 may be transmitted as thermal energy to a portion of the dermis layer in contact with the conductive material 200. The skin can be treated by the heat transmitted to the dermis layer of the skin.

The controller 140 may control the high-frequency generator 120.

The controller 140 may be connected to switches (not shown) arranged on an outer side surface of the handpiece case 110. A user may generate control signals by operating the switches.

The controller 140 may change at least one of the intensity, frequency, and treatment time of the high-frequency according to the condition of the skin to be treated or a treatment site.

Meanwhile, the high-frequency treatment device 100 may further include a power supply (not shown) configured to supply power. The power supply may be charged by a wired or wireless charging method.

Hereinafter, operations of the high-frequency treatment device 100 configured as described above will be described.

A practitioner may inject the conductive material 200 into the skin to be treated. In this case, the practitioner may use a syringe to inject the conductive material 200 into the skin at a depth of approximately 0.1 to 4 mm.

Once the injection of the conductive material 200 is completed, the practitioner may bring the close contact surface of the front end of the handpiece case 110 into close contact with the patient's skin while gripping the high-frequency treatment device 100.

The practitioner may operate the switches (not shown) disposed on the handpiece case 110 of the high-frequency treatment device 100 to operate the high-frequency generator 120.

The high-frequency generator 120 may generate a high-frequency current and transmit the generated high-frequency current to the coil part 130.

The coil part 130 may convert the high-frequency current output from the high-frequency generator 120 into an alternative magnetic field.

The alternative magnetic field generated by the coil part 130 may be transmitted to the conductive material 200 injected into the skin, which causes a current to flow in the conductive material 200.

The current can flow in the conductive material 200, which is injected into the skin, by the alternative magnetic field generated in the coil part 130. An eddy current loss and a hysteresis loss may occur due to the current flowing in the conductive material 200. The conductive material 200 may generate heat due to the eddy current loss and the hysteresis loss. The heat generated in the conductive material 200 may be transmitted as thermal energy to a portion of the dermis layer with which the conductive material 200 is in contact. The skin can be treated by the heat transmitted to the dermis layer of the skin.

That is, the conductive material 200 injected into the skin to be treated (affected area) may be heated due to the alternative magnetic field generated by the coil part 130 to output thermal energy as shown at points A in FIG. 6A. The thermal energy output from the conductive material 200 may coagulate the tissue in the dermis layer, and may regenerate and activate collagen.

In addition, the conductive material 200 injected into the affected area may be heated due to the alternative magnetic field generated by the coil part 130 to generate heat, and the generated heat can stimulate cancer cells, damaged cells, and aged cells in the affected area, which can lead to the removal or healing of these cancer cells, damaged cells, and aged cells in the affected area. At this point, a heating temperature or the like in the conductive material 200 may be determined by an intensity of the alternative magnetic field, an area of the conductive material 200, and a distance between the coil part 130 and the conductive material 200.

As described above, in the non-invasive high-frequency treatment system according to one embodiment of the present invention, a conductive material is injected into the skin to be treated, and heat is generated in the conductive material, which is injected into the skin, during a high-frequency treatment (procedure), so that the skin can be treated (or undergo a procedure) using high-frequency without inserting a needle into the skin, thereby eliminating skin burning or redness that may occur after the treatment (procedure).

Further, in the non-invasive high-frequency treatment system according to one embodiment of the present invention, electrical energy can be transmitted as thermal energy into the skin using high-frequency induction heating technology, thereby promoting skin tissue regeneration and enhancing skin cosmetic effects.

Further, in the non-invasive high-frequency treatment system according to one embodiment of the present invention, when a conductive material is inserted (injected) into a specific affected area in the human body and a strong alternative magnetic field is generated, only cancer cells in a desired affected area can be removed.

Further, in the non-invasive high-frequency treatment system according to one embodiment of the present invention, when a conductive material is inserted (injected) into a specific affected area in the human body and a strong alternative magnetic field is generated, cells in the affected area can be stimulated so that damaged or aged cells can be removed or healed, and the tissues of heat-sensitive cells can be further strengthened so that skin elasticity can be maintained, skin diseases can be alleviated, and the expansion and proliferation of potential cancer cells in the human body can be suppressed.

In a non-invasive high-frequency treatment system according to one embodiment of the present invention, a conductive material is injected into the skin to be treated, and heat is generated in the conductive material, which is injected into the skin, during a high-frequency treatment (procedure), so that the skin can be treated (or undergo a procedure) using high-frequency without inserting a needle into the skin, thereby eliminating skin burning or redness that may occur after the treatment (procedure).

Further, in a non-invasive high-frequency treatment system according to one embodiment of the present invention, electrical energy can be transmitted into the skin as thermal energy using high-frequency induction heating technology, thereby promoting skin tissue regeneration and enhancing skin cosmetic effects.

Further, in a non-invasive high-frequency treatment system according to one embodiment of the present invention, when a conductive material is inserted (injected) into a specific affected area in the human body and a strong alternative magnetic field is generated, only cancer cells in a desired affected area can be removed.

Further, in a non-invasive high-frequency treatment system according to one embodiment of the present invention, when a conductive material is inserted (injected) into a specific affected area in the human body and a strong alternative magnetic field is generated, cells in the affected area can be stimulated so that damaged or aged cells can be removed or healed, and the tissues of heat-sensitive cells can be further strengthened so that skin elasticity can be maintained, skin diseases can be alleviated, and the expansion and proliferation of potential cancer cells in the human body can be suppressed.

Meanwhile, the effects of the present invention are not limited to the effects described above, and various effects may be included within the scope that is apparent to those skilled in the art from the following description.

Although the embodiments of the present invention have been described with reference to the drawings, it would be understood that they are merely examples and various modifications and equivalents thereof may be made by one of ordinary skill in the art. Accordingly, the veritable technical scope of the present invention should be defined by the following claims.

## Claims

1. A non-invasive high-frequency treatment system comprising:
a conductive material injected into a skin to be treated; and
a high-frequency treatment device configured to generate a high-frequency current to transmit an alternative magnetic field to the conductive material,
wherein the high-frequency treatment device includes:
a high-frequency generator configured to output the high-frequency current;
a coil part configured to convert the high-frequency current output from the high-frequency generator into the alternative magnetic field; and
a controller configured to control the high-frequency generator, and
the conductive material generates heat due to the alternative magnetic field generated by the coil part.

2. The non-invasive high-frequency treatment system of claim 1, wherein the conductive material includes nanoparticles of a material having an electrical conductivity greater than or equal to a predetermined value.

3. The non-invasive high-frequency treatment system of claim 2, wherein the conductive material is in the form of at least one of hydrogel and collagen.

4. The non-invasive high-frequency treatment system of claim 2, wherein the conductive material converts a current induced by the alternative magnetic field into heat through an eddy current loss and a hysteresis loss.

5. The non-invasive high-frequency treatment system of claim 4, wherein the heat converted in the conductive material is transmitted as thermal energy to a portion of a dermis layer in contact with the conductive material, thereby treating the skin.

6. The non-invasive high-frequency treatment system of claim 1, wherein the coil part includes:
a substrate; and
a coil disposed on the substrate,
wherein the substrate is formed of a non-conductor, and has one surface serving as a close contact surface in contact with the skin of a user during a procedure and the other surface on which the coil is disposed.

7. The non-invasive high-frequency treatment system of claim 1, wherein the coil part is disposed on a front end of a handpiece case.

8. The non-invasive high-frequency treatment system of claim 1, wherein the controller changes at least one of an intensity, a frequency, and a treatment time of high-frequency according to a condition of the skin to be treated or a treatment site.

9. The non-invasive high-frequency treatment system of claim 1, wherein the high-frequency treatment device further includes a power supply that supplies power,
wherein the power supply is charged by a wired or wireless charging method.
